# EUROPEAN PATENT SPECIFICATION

(11) **EP 2 585 452 B1**
(45) Date of publication and mention of the grant of the patent: **23.09.2015**
(21) Application number: 11703429.8
(22) Date of filing: 03.02.2011
(51) Int. Cl.: C07D 413/12

(54) **PROCESS FOR THE PREPARATION OF PROPIONIC ACID DERIVATIVES**
VERFAHREN ZUR HERSTELLUNG VON PROPANSÄUREDERIVATEN
PROCÉDÉ POUR LA PRÉPARATION DE DÉRIVÉS D'ACIDE PROPIONIQUE

(30) Priority: 07.12.2009 EP 09178220
(43) Date of publication of application: 01.05.2013
(73) Proprietor: F.Hoffmann-La Roche AG, 4070 Basel (CH)
(72) Inventor: PUENTENER, Kurt, CH-4052 Basel (CH); SCALONE, Michelangelo, CH-4127 Birsfelden (CH)
(74) Representative: Weickmann & Weickmann
(86) International application number: PCT/EP2011/051610
(87) International publication number: WO 2011/070179

(56) References cited:
- EP-A1- 2 272 853
- WO-A1-2005/030764
- WO-A1-2010/108861
- LI SHEN ET AL: "Iridium-Catalyzed Enantioselective Hydrogenation of .alpha.,.beta.-Unsaturated Carboxylic Acids", JOURNAL OF THE AMERICAN CHEMICAL SOCIETY, AMERICAN CHEMICAL SOCIETY, US, vol. 130, no. 27, 9 July 2008 (2008-07-09) , pages 8584-8585, XP002580008, ISSN: 0002-7863, DOI: DOI:10.1021/JA802399V [retrieved on 2008-06-12]
- ZHU S -F ET AL: "Well-defined chiral spiro iridium/phosphine-oxazoline cationic complexes for highly enantioselective hydrogenation of imines at ambient pressure", JOURNAL OF THE AMERICAN CHEMICAL SOCIETY 20061004 US LNKD- DOI:10.1021/JA063444P, vol. 128, no. 39, 4 October 2006 (2006-10-04), pages 12886-12891, XP002627321, ISSN: 0002-7863

## Description

The present invention is concerned with a novel process for the preparation of (S)-2-methoxy-3-{4-[2-(5-methyl-2-phenyl-oxazol-4-yl)-ethoxy]-benzo[b]thiophen-7-yl}-propionic acid or a salt thereof.

The invention relates to a process for the preparation of a compound of formula (I) 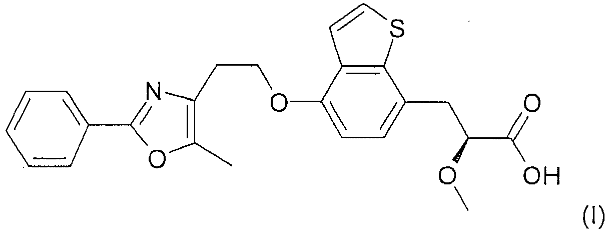 or a salt thereof, wherein a compound of formula (II) 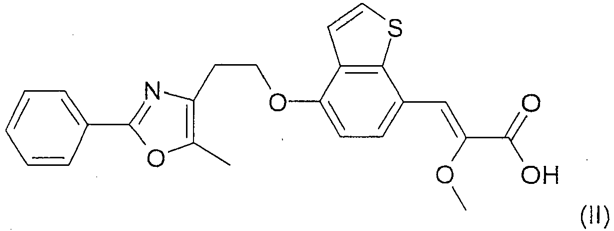 or a salt thereof is hydrogenated
(a) in the presence of a catalyst comprising iridium and a compound of formula (X) as defined below,

The compound of formula (I) is known in the art and is described for example in international application WO 02/092084. It is especially useful for the prophylaxis and/or treatment of diabetes mellitus type I and II.

Processes for the synthesis of the compound of formula (I) are disclosed in WO2005/030764 and WO2010/108861.

The process according to the invention allows the synthesis of the compound of formula (I) with high enantiomeric excess. It can be performed in dichloromethane and the use of complex solvent mixtures can be avoided. The process with the catalyst comprising iridium gives particularly high yield and high enantiomeric excess of the compound of formula (I). Furthermore, optically pure compound of formula (I) is obtained without the use of multiple crystallization of diastereomeric salts.

The term "catalyst" refers to a complex of iridium with a chiral ligand.

In such iridium complexes, iridium is preferably characterized by the oxidation number I.

The term "alkyl" refers to a branched or straight chain monovalent alkyl radical of one to eight carbon atoms, preferably one to four carbon atoms. This term is further exemplified by such radicals as methyl, ethyl, *n*-propyl, *iso*-propyl, *iso*-butyl, *n*-butyl, *tert-*butyl and the like with methyl, *tert*-butyl and *iso*-propyl being preferred.

The term "alkoxy" refers to the group alkyl-O-. A preferred alkoxy group is methoxy.

The term "cycloalkyl" refers to a monovalent carbocyclic radical of 3 to 10 carbon atoms, preferably 3 to 6 carbon atoms, such as cyclopropyl, cyclobutyl, cyclopentyl, or cyclohexyl. Cyclohexyl is a preferred cycloalkyl.

The term "aryl" relates to the phenyl or naphthyl group, preferably the phenyl group, which can optionally be mono- or multiply-substituted, particularly mono-, di- or tri-substituted by halogen, hydroxy, CN, CF₃, NO₂, NH₂, N(H, alkyl), N(alkyl)₂, carboxy, aminocarbonyl, alkyl, alkoxy, phenyl and/or phenyloxy. Preferred substituents are halogen, alkyl, CF₃ and alkoxy, particularly alkyl, CF₃ and alkoxy.

The term "heteroaryl" refers to an aromatic 5- or 6-membered ring which can comprise 1, 2 or 3 atoms selected from nitrogen, oxygen and/or sulphur such as furyl, pyridyl, 1,2-, 1,3- and 1,4-diazinyl, thienyl, isoxazolyl, oxazolyl, imidazolyl, or pyrrolyl. The term "heteroaryl" further refers to bicyclic aromatic groups comprising two 5- or 6-membered rings, in which one or both rings can comprise 1, 2 or 3 atoms selected from nitrogen, oxygen or sulphur such as e.g. indole or quinoline, or partially hydrogenated bicyclic aromatic groups such as e.g. indolinyl. A heteroaryl group may have a substitution pattern as described earlier in connection with the term "aryl". Preferred heteroaryl groups are 2-thienyl and 2-furyl. 2-Furyl is particularly preferred.

The term "halide" refers to a halogen atom bearing a negative charge such as fluoride, chloride, bromide and iodide.

The term "pharmaceutically acceptable salts" embraces salts of the compound of formula (I) with pharmaceutically acceptable bases such as alkali salts, e.g. Na- and K-salts, alkaline earth salts, e.g. Ca- and Mg-salts, and ammonium or alkyl-substituted ammonium salts, such as e.g. trimethylammonium salts. A preferred pharmaceutically acceptable salt of the compound of formula (I) is the sodium salt.

The term "η⁵" means eta5 as used normaly in coordination chemistry. It indicates the number of electrons shared between a metal center and a ligand in a coordination compound or complex.

The invention relates to a process as defined above wherein the catalyst comprises iridium and a compound of formula (X) 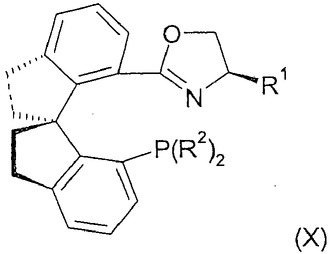 wherein
R¹ is alkyl, aryl or arylalkyl;
R² is aryl.
R¹ is preferably alkyl, phenyl or benzyl, more preferably alkyl or benzyl.

In particular, a process as defined above wherein R¹ is *iso*-propyl, phenyl or benzyl is preferred. More preferably, R¹ is *iso*-propyl or benzyl.

Also preferred is a process as defined above wherein R² is phenyl or phenyl substituted with one or two alkyl.

Moreover, preferred is a process according to the invention wherein R² is phenyl, 3,5-di-methylphenyl or 3,5-di-*tert*-butyl-phenyl.

Still further preferred is a process according to the invention wherein the compound of formula (X) is
(Sₐ,R)-7-[4,5-Dihydro-4-benzyloxazol-2-yl]-7'-diphenylphosphino-1,1 '-spirobiindane;
(Sₐ,R)-7-[4,5-Dihydro-4-benzyloxazol-2-yl]-7'-di(3,5-di-methylphenyl)phosphino-1,1'-spirobiindane;
(Sₐ,R)-7-[4,5-Dihydro-4-benzyloxazol-2-yl]-7'-di(3,5-di-tert-butylphenyl) phosphino-1,1'-spirobiindane;
(Sₐ,R)-7-[4,5-Dihydro-4-phenyloxazol-2-yl]-7'-di(3,5-di-tert-butylphenyl) phosphino-1,1'-spirobiindane; or
(Sₐ,R)-7-[4,5-Dihydro-4-isopropyloxazol-2-yl]-7'-di(3,5-di-tert-butylphenyl) phosphino-1,1'-spirobiindane.

Moreover, preferred is a process as defined above wherein the catalyst is Ir(L¹)(L²)ₙY
wherein
L¹ is a compound of formula (X) as defined above;
L² is cyclooctene, 1,5-cyclooctadiene, ethylene, 1,5-hexadiene or norbornadiene;
Y is chloride, iodide, bromide, fluoride, trifluoroacetate, tetrafluoroborate, tetrakis[3,5-bis(trifluoromethyl)phenyl]borate, tetraphenylborate, hexafluoroantimonate, hexafluorophosphate, triflate, mesylate, perchlorate, perbromate, periodate, nitrate, hydrogen sulfate or acetylacetonate; and
n is 1 or 2.
Y is preferably chloride, tetrafluoroborate, hexafluorophosphate or tetrakis[3,5-bis(trifluoromethyl)phenyl]borate, more preferably tetrafluoroborate or tetrakis[3,5-bis(trifluoromethyl)phenyl]borate.
n is preferably 1.

Further particularly preferred is a process according to the invention wherein the catalyst is
[Ir((Sₐ,R)-7-[4,5-dihydro-4-benzyloxazol-2-yl]-7'-di(3,5-di-tert-butylphenyl) phosphino-1,1'-spirobiindane)(1,5-cyclooctadiene)][tetrakis[3,5-bis(trifluoromethyl)phenyl]borate];
[Ir((Sₐ,R)-7-[4,5-dihydro-4-benzyloxazol-2-yl]-7'-di(3,5-di-tert-butylphenyl) phosphino-1,1'-spirobiindane)(1,5-cyclooctadiene)][tetrafluoroborate];
[Ir((Sₐ,R)-7-[4,5-dihydro-4-benzyloxazol-2-yl]-7'-di(3,5-di-tert-butytphenyl) phosphino-1,1'-spirobiindane)(1,5-cyclooctadiene)][trifluoromethanesulfonate];or
[Ir((Sₐ,R)-7-[4,5-dihydro-4-benzyloxazol-2-yl]-7'-di(3,5-di-tert-butylphenyl) phosphine-1,1'-spirobiindane)(1,5-cyclooctadiene)][chloride].

According to the invention, the compound of formula (II) can be hydrogenated under a pressure of hydrogen gas.

When an iridium catalyst is used, the process is preferably carried out at a temperature of 10 to 120°C, more preferably 40 to 100°C, particularly preferably 60 to 80°C.

When a catalyst comprising iridium is used, the process is-preferably carried out in a solvent selected from alcohols, fluorinated alcohols, tetrahydrofuran, methyl-tetrahydrofuran, dichloromethane, dialkyl ethers, aromatic solvents such as benzene, toluene, CF₃-C₆H₅, mono- and poly-fluorinated aromatic solvents and mixtures thereof, more preferred in methanol, tetrahydrofuran, dichloromethane and mixtures thereof, most preferably in methanol/tetrahydrofuran 3:2.

When a catalyst comprising iridium is used, the process is preferably carried out under a hydrogen pressure range of 1 to 200 bar, more preferably 10 to 100 bar, particularly preferably 40 to 60 bar. When the ligand of formula (III) of (S,S) configuration is used, preferred is a pressure of 10 bar. When the.ligand of formula (III) of (Sₐ,R) configuration is used, preferred is a pressure of 30 bar.

When a catalyst comprising iridium is used, the substrate-to-catalyst ratio (mol/mol) is preferably 10 to 50000, more preferably between 100 and 10000, particularly preferably between 1000 and 5000.

The preferred (S) configuration of the compound of formula (I) has been obtained with the ligands disclosed in the tables in the experimental part. Should a chiral ligand or catalyst afford preferentially the compound of formula (I) with the (R) configuration, it is clear that the ligand or catalyst with the opposite configuration should be used in order to obtain the compound of formula (I) with the (S) configuration. Both enantiomers of the chiral ligands are equally well accessible.

The invention also relates to a compound of formula (I) as defined above or a salt thereof obtained by a process according to the invention.

Furthermore, the invention also relates to the use of a catalyst as defined above for the preparation of a compound of formula (I) as defined above.

The catalysts for use in the process of the present invention may be prepared by reacting a compound of formula [Ir(L)Cl]₂, [Ir(L)₂]BARF or [Ir(L)₂]BF₄ where L denotes a neutral ligand, e.g. COD with the desired ligand of formula (X) in an appropriate solvent, such as e.g. dichloromethane or methanol. The catalyst may be used after isolation or as prepared in situ. The compounds [Ir(COD)Cl]₂ and [Ir⁺ (COD)₂]BF₄ are either commercially available, e.g. from Strem Chemicals Inc., Newburgport, Mass. USA or can be prepared according to to methods known per se, e.g. J. Herde et al., Inorg. Syn. 1974, 18-20 or M. Green et al., J. Chem. Soc. 1971, 2334-2337.

The term "neutral ligand" as used herein denotes a readily exchangeable ligand such as an olefin, e.g. ethylene, propylene, cyclooctene, 1,5-hexadiene, norbornadiene, 1,5-cyclooctadiene, a nitrile such as acetonitrile or benzonitrile, or also a solvent such as e.g. tetrahydrofuran, toluene etc. Where more than one such ligand is present, these can also be different from each other. A preferred neutral ligand is cyclooctadiene.

### Examples

### Abbreviations

η⁵-2,4-DMP = η⁵-2,4-dimethylpentadienyl,
THF = tetrahydrofuran,
NCMe = acetonitrile,
TFA = trifluoroacetic acid,
COD = 1,5-cyclooctadiene,
BARF = tetrakis[3,5-bis(trifluoromethyl)phenyl]borate,
r.t. = room temperature,
S/C = substrate-to-catalyst ratio (mol/mol),
HPLC = high pressure liquid chromatography,
ee = enantiomeric excess = [(S)-(R)]/[(S)+(R)].
DBT = DTB = 3,5-di-tert.-butylphenyl
Triflate = trifluoromethanesulfonate

All ferrocenyl-diphosphine ligands are commercially available from Solvias AG, CH-4002 Basel. The ruthenium complexes are commercially available from Umicore AG, D-63457 Hanau-Wolfgang or can be prepared according to O. Briel et al. in "Catalysis of Organic Reactions", 2009, 203, CRC Press, Boca Raton. The oxazoline-monophosphine ligands (SIPHOX ligands) and their corresponding iridium complexes are commercially available from Nankai University, Tianjin 300071 China or can be prepared according to Q.L. Zhou et al. J. Am. Chem. Soc. 2008, 130, 8584. Xyl-Skewphos and 3,5-tBu-MeOBIPHEP are commercially available from Solvias AG, CH-4002 Basel. TRIFER is commercially available from Phoenix Chemicals, 34 Thursby Rod., Bromborough, Wirral CH62, 3PW, United Kingdom (UK) or can be prepared according to P. McCormack et al. Angew. Chem. Int. Ed. 2007, 46, 4141-44.

The atom numbering of SIPHOX ligands is shown below:

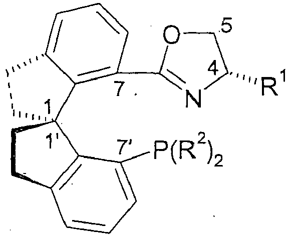

The asymmetric configuration of the (Sₐ,R) SIPHOX ligand is shown below:

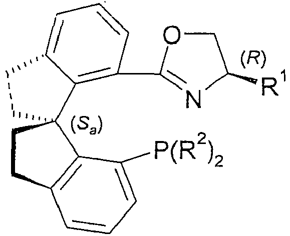

The (Sₐ,S) or (Sₐ,R) configuration of the SIPHOX ligand may also be noted (S,S) or (S,R) respectively.

### Chiral phosphorus Ligands

| ***Acronyms*** | ***Chemical Name*** |
|---|---|
| Ph-Bn-SIPHOX | 7-[4,5-Dihydro-4-benzyloxazol-2-yl]-7'-diphenylphosphino-1,1'-spirobiindane |
| Xyl-Bn-SIPHOX | 7-[4,5-Dihydro-4-benzyloxazol-2-yl]-7'-di(3,5-di-methylphenyl)phosphino-1,1'-spirobiindane |
| DBT-Bn-SIPHOX | 7-[4,5-Dihydro-4-benzyloxazol-2-yl]-7'-di(3,5-di-tert-butylphenyl) phosphino-1,1'-spirobiindane |
| DBT-Ph-SIPHOX | 7-[4,5-Dihydro-4-phenyloxazol-2-yl]-7'-di(3,5-di-tert-butyiphenyl) phosphino-1,1'-spirobiindane |
| DBT-iPr-SIPHOX | 7-[4,5-Dihydro-4-isopropyloxazol-2-yl]-7'-di(3,5-di-tert-butylphenyl) phosphino-1,1'-spirobiindane |
| DBT-H-SIPHOX | 7-[4,5-Dihydrooxazol-2-yl]-7'-di(3,5-di-tert-butylphenyl)phosphino-1,1'-spirobiindane |
| TRIFER | 1,1'-Bis-[((1-N,N-dimethylamino)ethylferrocenyl) (phenylphosphi-no)]ferrocene |
| Xyl-Skewphos | [1,3-Dimethyl-1,3-propanediyl]bis[di-(3,5-dimethylphenyl)phosphine] |
| PPF-PtBu₂ | 1-[2-(Diphenylphosphino)ferrocenyl] ethyldi-tert.- butylphosphine |
| Cy₂PF-PtBu₂ | 1-[2-(Dicyclohexylphosphino)ferrocenyl]ethyldi-tert.-butylphosphine |
| (4-CF₃Ph)₂PF-PtBu₂ | 1-[2-(Di-(4-trifluoromethylphenyl) phosphino)ferrocenyl]ethyldi-tert.-butyl phosphine |
| (3,5-Me₂-4-MeOPh)₂PF-PtBu₂ | 1-[2-(Di-(3,5-dimethyl-4-methoxy phenyl)phosphino)ferrocenyl]ethyldi-tert.-butylphosphine |
| 2-Fur₂PF-PtBu₂ | 1-[2-(Di-2-furylphosphino)ferrocenyl]ethyldi-tert.-butylphosphine |
| NMe₂-PPh₂-Mandyphos | 2,2'-Bis(α-N,N-dimethylaminophe-nylmethyl)-1,1'-bis(diphenylphosphino) ferrocene |
| NMe₂-P(3,5-Me-4-MeOPh)₂-Mandyphos | 2,2'-Bis(α-N,N-dimethylaminophenylmethyl)-1,1'-bis[di(3,5-dimethyl-4-methoxyphenyl)-phosphinolferrocene |
| PPPhCHNMe₂-F-PP | 1-Diphenylphosphino-2-[α-(N,N-dimethylamino)-o-diphenylphosphinophenyl) methyl]ferrocene |
| PPPhFCHCH₃-P(3,5-CF₃Ph)₂ | 1-[2-(2'-Diphenylphosphinophenyl) ferrocenyl]-ethyldi(bis-3,5-trifluoromethyl phenyl)phosphine |
| Cy₂PPhFCH CH₃P(3,5-CF₃Ph)₂ | 1-[2-(2'-Dicyclohexylphosphino phenyl)ferrocenyl]ethyldi(bis-3,5-trifluoromethylphenyl)phosphine |
| PPPhFCHCH₃-P(Norbornyl)₂ | 1-[2-(2'-Diphenylphosphinophenyl) ferrocenyl]ethyldi-(2-norbornyl)phosphine |

### Synthesis of Iridium Mental Complexes: Examples 1a-1h

### Example 1.a: Preparation of [Ir((S,S)-Xyl-Skewphos)(COD)]BF₄

A 25-ml Schlenk tube was charged with 100 mg of (S,S)-Xyl-Skewphos (0.18 mmol), 60 mg of [Ir(COD)Cl]₂ (0.09 mmol) and 5 ml of dichloromethane. To the formed dark red solution, 35 mg of silver tetrafluoroborate (0.18 mmol) was added in two protions and the resulting suspension was stirred for 2 hours at r.t. The reaction mixture was filtered over dicalite speedex and the filter cake was washed with 6 ml of dichloromethane. The combined filtrates were rotary evaporated to dryness (50°C/5 mbar). The formed crude product was washed with 8 ml of hexane and dried over high vacuum to afford 563 mg (85%) of [Ir((S,S)-Xyl-Skewphos)(COD)]BF₄ as a red solid. FT-MS: 853.4 m/z [Ir((S,S)-Xyl-Skewphos) (COD)]⁺, ³¹P-NMR (CDCl₃): 14.6 ppm (s).

### Example 1.b: Preparation of [Ir((S,R,R)-Trifer)(COD)]BARF

A 100-ml Schlenk tube was charged with 400 mg of (S,R,R)-TRIFER (0.44 mmol), 584 mg of Ir(COD)₂]BARF (0.46 mmol) and 40 ml of methanol. The formed orange solution was stirred for 5 hours at r.t. Then, 12 ml of water was added and the formed crystals were filtered off. The filter cake was washed with 32 ml of a mixture of methanol/water (4:1) and dried over high vacuum to afford 804 mg (88%) of [Ir((S,R,R)-TRIFER)(COD)]BARF as orange crystals. FT-MS: 1213.2 m/z [Ir((S,R,R)-TRIFER)(COD)]⁺. ³¹P-NMR (CDCl₃): 6.2 ppm (s).

### Example 1.c: Preparation of [Ir((Sₐ,R)-DBT-Bn-SIPHOX)(COD)]BARF

A 25-ml Schlenk tube was charged with 100 mg of (Sₐ,R)-DBT-Bn-SIPHOX (0.127 mmol), 168 mg of [Ir(COD)₂]BARF (0.132 mmol) and 10 ml of methanol. To the formed yellow solution was stirred for 2 hours at r.t., then the reaction mixture rotary evaporated to dryness (50°C/5 mbar). The residue was dissolved in 5 ml of methanol. 0.5 ml of water was added and the formed yellow suspension was stirred for 30 min at r.t.. The crystals were filtered off, washed with 3.5 ml of MeOH / water (6:1) and dried over high vacuum to afford 189 mg (76%) of [Ir((Sₐ,R)-DBT-Bn-SIPHOX)(COD)]BARF as an organe solid. FT-MS: 1088.5 m/z [Ir((Sₐ,R)-DBT-Bn-SIPHOX)(COD)]⁺, ³¹P-NMR (CDCl₃): 16.5 ppm (s).

### Example 1.d: Preparation of [Ir((Sₐ,R)-DBT-Bn-SIPHOX)(COD)]BF₄

A 25-ml Schlenk tube was charged with 56 mg of (Sₐ,R)-DBT-Bn-SIPHOX (0.070 mmol), 24 mg of [Ir(COD)Cl]₂ (0.035 mmol) and 5 ml of dichloromethane. To the formed orange solution, 14 mg of silver tetrafluoroborate (0.071 mmol) was added and the resulting orange suspension was stirred for 2 hours at r.t. The reaction mixture was filtered over dicalite speedex and the filter cake was washed with 9 ml of dichloromethane. The combined filtrates were rotary evaporated to dryness (50°C/5 mbar). The formed crude product was washed with 11 ml of hexane and dried over high vacuum to afford 69 mg (83%) of [Ir((Sₐ,R)-DBT-Bn-SIPHOX) (COD)]BF₄ as an orange solid. FT-MS: 1088.5 m/z [Ir((Sₐ,R)-DBT-Bn-SIPHOX) (COD)]⁺, ³¹P-NMR (CDCl₃): 16.6 ppm (s).

### Example 1.e: Preparation of [Ir((Rₐ,S)-DBT-Bn-SIPHOX)(COD)]OTf

A 25-ml Schlenk tube was charged with 100 mg of (Rₐ,S)-DBT-Bn-SIPHOX (0.127 mmol), 44 mg of [Ir(COD)Cl]₂ (0.505 mmol) and 4 ml of dichloromethane. To the formed orange solution, 34 mg of silver trifluoromethanesulfonate (0.130 mmol) was added and the resulting orange suspension was stirred for 2 hours at r.t. The reaction mixture was filtered over dicalite speedex and the filter cake was washed with 6 ml of dichloromethane. The combined filtrates were rotary evaporated to dryness (50°C/5 mbar). The formed crude product was washed with 11 ml of hexane and dried over high vacuum to afford 134 mg (85%) of [Ir((Rₐ,S)-DBT-Bn-SIPHOX)(COD)]OTf as an orange solid. FT-MS: 1088.5 m/z [Ir((Rₐ,S)-DBT-Bn-SIPHOX)(COD)]⁺, ³¹P-NMR (CDCl₃): 16.6 ppm (s).

### Example 1.f: Preparation of [Ir((S,S)-DBT-Bn-SIPHOX)(COD)]BF₄

A 25-ml Schlenk tube was charged with 100 mg of (S,S)-DBT-Bn-SIPHOX (0.127 mmol), 43 mg of [Ir(COD)Cl]₂ (0.063 mmol) and 5 ml of dichloromethane. To the formed orange solution, 26 mg of silver tetrafluoroborate (0.131 mmol) was added and the resulting orange suspension was stirred for 2 hours at r.t. The reaction mixture was filtered over dicalite speedex and the filter cake was washed with 6 ml of dichloromethane. The combined filtrates were rotary evaporated to dryness (50°C/5 mbar). The formed crude product was washed with 8 ml of hexane and dried over high vacuum to afford 148 mg (99%) of [Ir((S,S)-DBT-Bn-SIPHOX) (COD)]BF₄ as an orange solid. FT-MS: 1088.5 m/z [Ir((S,S)-DBT-Bn-SIPHOX) (COD)]⁺, ³¹P-NMR (CDCl₃): 16.0 ppm (s).

### Example 1.g: Preparation of [Ir((S,S)-DBT-Bn-SIPHOX)(COD)]BF₄

A 25-ml Schlenk tube was charged with 200 mg of (S,S)-DBT-Bn-SIPHOX (0.254 mmol), 87 mg of [Ir(COD)Cl]₂ (0.128 mmol) and 5 ml of dichloromethane. To the formed orange solution, 43 mg of sodium tetrafluoroborate (0.384 mmol) was added and the resulting orange suspension was stirred for 5 hours at r.t. The reaction mixture was filtered over dicalite speedex and the filter cake was washed with 8 ml of dichloromethane. The combined filtrates were rotary evaporated to dryness (50°C/5 mbar). The formed crude product was washed with 11 ml of hexane and dried over high vacuum to afford 259 mg (87%) of [Ir((S,S)-DBT-Bn-SIPHOX)(COD)]BF₄ as an orange solid. FT-MS: 1088.5 m/z [Ir((S,S)-DBT-Bn-SIPHOX)(COD)]⁺, ³¹P-NMR (CDCl₃): 16.0 ppm (s).

### Example 1.h: Preparation of [Ir((Rₐ,S)-DBT-Bn-SIPHOX)(COD)]BF₄

A 25-ml Schlenk tube was charged with 40 mg of (Rₐ,S)-DBT-Bn-SIPHOX (0.058 mmol), 18 mg of [Ir(COD)Cl]₂ (0.026 mmol) and 4 ml of dichloromethane. To the formed orange solution, 9 mg of sodium tetrafluoroborate (0.076 mmol) was added and the resulting orange suspension was stirred for 3 hours at r.t. The reaction mixture was filtered over dicalite speedex and the filter cake was washed with 6 ml of dichloromethane. The combined filtrates were rotary evaporated to dryness (50°C/5 mbar). The formed crude product was washed with 11 ml of hexane and dried over high vacuum to afford 51 mg (86%) of [Ir((Rₐ,S)-DBT-Bn-SIPHOX)(COD)]BF₄ as an orange solid. FT-MS: 1088.5 m/z [Ir((Rₐ,S)-DBT-Bn-SIPHOX)(COD)]⁺, ³¹P-NMR (CDCl₃): 16.6 ppm (s).

### Synthesis of 2-Methoxy-3-{4-[2-(5-methyl-2-phenyl-oxazol-4-yl)-ethoxy]-benzo[b]thiophen-7-yl}-propionic acid via asymmetric hydrogenation of (Z)-2-methoxy-3-{4-[2-(5-methyl-2-phenyl-oxazol-4-yl)-ethoxy]-benzo[b]thiophen-7-yl}-acrylic acid: Examples 2- 11

### Example 2.1

In a glove box (O₂ content ≤ 2 ppm ), a 185- ml stainless steel autoclave was charged with 2.00 g of (Z)-2-methoxy-3-{4-[2-(5-methyl-2-phenyl-oxazol-4-yl)-ethoxy]-benzo[b]thiophen-7-yl}-acrylic acid (4.59 mmol), 35.9 mg of [lr((S,S)-DBT-Bn-SIPHOX)(COD)]BARF (0.018 mmol, S/C 250), 24 ml of methanol, 16 ml of tetrahydrofuran and 0.12 ml of (S)-1-phenylethylamine (0.93 mmol). The autoclave was sealed and the hydrogenation was run at 60°C under 30 bar of hydrogen. After 16 h the autoclave was opened and the yellowish solution was rotary evaporated to dryness (50°C/5 mbar) to afford crude (S)-2-methoxy-3-{4-[2-(5-methyl-2-phenyl-oxazol-4-yl)-ethoxy]-benzo[b]thiophen-7-yl}-propionic acid (Acid I) as a white solid with a chemical purity of 99.6 % (>99.9% conversion) and an enantiomeric purity of 99.5%.

HPLC method for chemical purity (area-%, (S)-phenylethylamine not included): YMC-Pack Pro C18, 150 x 4.6 mm; mobile phase A: mobile phase A: water with 0.1 % TFA, B: NCMe with 0.1 % TFA, 22°C, 2 ml/min, isocratic A/B 51/49% during 10 min, gradient from 51/49% to 5/95% within 10 min and 5 min at 5/95%, 285 nm. Retention times: 11.2 min (S)- and (R)-2-methoxy-3-{4-[2-(5-methyl-2-phenyl-oxazol-4-yl)-ethoxy]-benzo[b]thiophen-7-yl}-propionic acid; 12.4 min (E)-2-methoxy-3-{4-[2-(5-methyl-2-phenyl-oxazol-4-yl)-ethoxy]-benzo[b]thiophen-7-yl}-acrylic acid; 14.0 min (Z)-2-methoxy-3-{4-[2-(5-methyl-2-phenyl-oxazol-4-yl)-ethoxy]-benzo[b]thiophen-7-yl}-acrylic acid.

HPLC method for ee determination (area-%): Chiralpak-ADH column, 25 cm x 4.6 mm, 85% heptane / 10% ethanol with 0.4% trifluoroacetic acid, flow ,0.7 ml/min, 30°C, 270 nm. Retention times: 22.4 min (R)-2-methoxy-3-{4-[2-(5-methyl-2-phenyl-oxazol-4-yl)-ethoxy]-benzo[b]thiophen-7-yl}-propionic acid; 26.3 min (S)-2-methoxy-3-{4-[2-(5-methyl-2-phenyl-oxazol-4-yl)-ethoxy]-benzo[b]thiophen-7-yl}-propionic acid.

### Example 2.2

In a glove box (O₂ content ≤ 2 ppm ), a 185- ml stainless steel autoclave was charged with 2.00 g of (Z)-2-methoxy-3-{4-[2-(5-methyl-2-phenyl-oxazol-4-yl)-ethoxy]-benzo[b]thiophen-7-yl}-acrylic acid (4.59 mmol), 2.70 mg of [Ir((Sₐ,R)-DBT-Bn-SIPHOX)(COD)]BF₄ (0.0023 mmol, S/C 2'000), 24 ml of methanol, 16 ml of tetrahydrofuran and 0.12 ml of (S)-1-phenylethylamine (0.92 mmol). The autoclave was sealed and the hydrogenation was run under 30 bar of hydrogen at 60°C for 20 h and subsequently at 80°C for 2 h. Then the autoclave was opened and the yellowish solution was rotary evaporated to dryness (50°C/5 mbar) to afford crude (S)-2-methoxy-3-{4-[2-(5-methyl-2-phenyl-oxazol-4-yl)-ethoxy]-benzo[b]thiophen-7-yl}-propionic acid (Acid I) as a white solid with a chemical purity of 89.9 % (>99.9% conversion) and an enantiomeric purity of 99.8%. The crude product was dissolved in 50 ml of ethyl acetate. 10 ml of water and 3 ml of 2M aqueous HCl were added and the biphasic mixture was stirred at 55°C for 15 min. The organic layer was separated, the aqueous layer extracted with 20 ml of ethyl acetate and the combined organic layers stirred over 0.5 g of carcoal (Darko KB) at r.t. for 2h. After filtration over celite, the colorless solution was dried over 3 g of sodium sulfate and evaporated to dryness (40°C/10 mbar). The crude product was dissolved in 50 ml of isopropyl acetate at reflux (oil bath temp. 100°C) and allowed to cool to room temperature whereby crystallization started sponanously. The formed crystals were filtered off, washed with 10 ml of isopropyl acetate and dried at 75°C/500mbar for 4 h to yield 1.60 g (79%) of pure (S)-2-methoxy-3-{4-[2-(5-methyl-2-phenyl-oxazol-4-yl)-ethoxy]-benzo[b]thiophen-7-yl}-propionic acid (Acid I) as a white crystals with a chemical purity of 99.6 % and an enantiomeric purity of 99.8%ee.

### Examples 3.1-3.4

In an analogous manner to Example 2 the following hydrogenations were performed at 60°C under 30 bar of hydrogen (reaction time: 16 h) using iridium complexes of general formula [Ir(Phosphorous Ligand)(COD)]BARF as catalysts to afford crude 2-methoxy-3-{4-[2-(5-methyl-2-phenyl-oxazol-4-yl)-ethoxy]-benzo[b]thiophen-7-yl}-propionic acid (Acid I) as listed in Table 1.

**Table 1**

| **Exp. No.** | **Phosphorus Ligand** | **Conv. [%]** | **Acid I Purity [%]** | **Acid I Ee [%]** / **Configuration** |
|---|---|---|---|---|
| 3.1 | (S,S)-Xyl-Bn-SIPHOX | 99.8 | 97.6 | 88.9/S |
| 3.2 | (S,S)-DBT-Ph-SIPHOX | 99.9 | 99.4 | 98.0/S |
| 3.3 | (S,S)-DBT-iPr-SIPHOX | >99.9 | 99.3 | 99.3/S |
| 3.4 | (S)-DBT-H-SIPHOX | >99.9 | 98.3 | 99.3/S |

### Example 4

In a glove box (O₂ content ≤ 2 ppm), a 185- ml stainless steel autoclave was charged with 2.00 g of (Z)-2-methoxy-3-{4-[2-(5-methyl-2-phenyl-oxazol-4-yl)-ethoxy]-benzo[b]thiophen-7-yl}-acrylic acid (4.59 mmol), 8.96 mg of [lr((S,S)-DBT-Bn-SIPHOX)(COD)]BARF (0.0046 mmol, S/C 1'000), 24 ml of methanol, 16 ml of tetrahydrofuran and 0.12 ml of (S)-1-phenylethylamine (0.93 mmol). The autoclave was sealed and the hydrogenation was run at 60°C under 30 bar of hydrogen. After 16 h the autoclave was opened and the yellowish solution was rotary evaporated to dryness (50°C/5 mbar) to afford the crude (S)-2-methoxy-3-{4-[2-(5-methyl-2-phenyl-oxazol-4-yl)-ethoxy]-benzo[b]thiophen-7-yl}-propionic acid (Acid I) as a white solid with a chemical purity of 99.2 % (99.8% conversion) and an enantiomeric purity of 99.3%.

### Examples 5.1-5.2

In an analogous manner to Example 4 the following hydrogenations were performed at 60°C under 30 bar of hydrogen (reaction time: 16 h) using iridium complexes of general formula [Ir(Phosphorus Ligand)(COD)]BARF as catalysts to afford crude 2-methoxy-3-{4-[2-(5-methyl-2-phenyl-oxazol-4-yl)-ethoxy]-benzo[b]thiophen-7-yl}-propionic acid (Acid I) as listed in Table 2.

**Table 2**

| **Exp. No.** | **Phosphorus Ligand** | **Conv. [%]** | **Acid I Purity [%]** | **Acid I Ee [%] / Configuration** |
|---|---|---|---|---|
| 5.1 | (S,S)-DBT-iPr-SIPHOX | 99.9 | 99.5 | 98.7/S |
| 5.2 | (S)-DBT-H-SIPHOX | 99.9 | 98.1 | 99.3/S |

### Example 6.1

In a glove box (O₂ content ≤ 2 ppm), a 185- ml stainless steel autoclave was charged with 2.00 g of (Z)-2-methoxy-3-{4-[2-(5-methyl-2-phenyl-oxazol-4-yl)-ethoxy]-benzo[b]thiophen-7-yl}-acrylic acid (4.59 mmol), 4.48 mg of [Ir((S,S)-DBT-Bn-SIPHOX)(COD)]BARF (0..0023 mmol, S/C 2'000), 24 ml of methanol, 16 ml of tetrahydrofuran and 0.12 ml of (S)-1-phenylethylamine (0.93 mmol). The autoclave was sealed and the hydrogenation was run at 60°C for 20 h and subsequently 80°C for 2 h under 30 bar of hydrogen. After the autoclave was opened and the yellowish solution was rotary evaporated to dryness (50°C/5 mbar) to afford crude (S)-2-methoxy-3-{4-[2-(5-methyl-2-phenyl-oxazol-4-yl)-ethoxy]-benzo[b]thiophen-7-yl}-propionic acid (Acid I) as a white solid with a chemical purity of 99.2 % (>99.9% conversion) and an enantiomeric purity of 99.4%.

### Example 6.2

In a glove box (O₂ content ≤ 2 ppm), a 185-ml stainless steel autoclave was charged with 2.00 g of (Z)-2-methoxy-3-{4-[2-(5-methyl-2-phenyl-oxazol-4-yl)-ethoxy]-benzo[b]thiophen-7-yl}-acrylic acid (4.59 mmol), 4.48 mg of [Ir((Sₐ,R)-DBT-Bn-SIPHOX)(COD)]BARF (0.0023 mmol, S/C 2'000), 24 ml of methanol, 16 ml of tetrahydrofuran and 0.12 ml of (S)-1-phenylethylamine (0.92 mmol). The autoclave was sealed and the hydrogenation was run under 30 bar of hydrogen at 60°C for 20 h and subsequently at 80°C for 2 h. Then the autoclave was opened and the yellowish solution was rotary evaporated to dryness (50°C/5 mbar) to afford crude (S)-2-methoxy-3-{4-[2-(5-methyl-2-phenyl-oxazol-4-yl)-ethoxy]-benzo[b]thiophen-7-yl}-propionic acid (Acid I) as a white solid with a chemical purity of 99.0 % (>99.9% conversion) and an enantiomeric purity of 99.8%.

### Example 7

In a glove box (O₂ content ≤ 2 ppm), a 185- ml stainless steel autoclave was charged with 2.00 g of (Z)-2-methoxy-3-{4-[2-(5-methyl-2-phenyl-oxazol-4-yl)-ethoxy]-benzo[b]thiophen-7-yl}-acrylic acid (4.59 mmol), 8.96 mg of [Ir((S,S)-DBT-Bn-SIPHOX)(COD)]BARF (0.0046 mmol, S/C 1'000), 24 ml of methanol, 16 ml of tetrahydrofuran and 0.12 ml of (S)-1-phenylethylamine (0.93 mmol). The autoclave was sealed and the hydrogenation was run at 60°C for 8 h and subsequently 80°C for 2 h under 30 bar of hydrogen. After the autoclave was opened and the yellowish solution was rotary evaporated to dryness (50°C/5 mbar) to afford 2.24 g of the crude (S)-2-methoxy-3-{4-[2-(5-methyl-2-phenyl-oxazol-4-yl)-ethoxy]-benzo[b]thiophen-7-yl}-propionic acid (Acid I) as a white solid with a chemical purity of 99.2 % (>99.9% conversion) and an enantiomeric purity of 99.2%. The crude product was dissolved in 50 ml of ethyl acetate. 10 ml of water and 3 ml of 2M aqueous HCl were added and the biphasic mixture was stirred at 55°C for 15 min. The organic layer was separated, the aqueous layer extracted with 20 ml of ethyl acetate and the combined organic layers stirred over 0.5 g of carcoal (Darko KB) at r.t. for 30 min. After filtration over celite, the colorless solution was dried over 3 g of sodium sulfate and evaporated to dryness (40°C/10 mbar). The crude product was dissolved in 50 ml of isopropyl acetate at reflux (oil bath temp. 100°C) and allowed to cool to room temperature whereby crystallization started sponanously. The formed crystals were filtered off, washed with 10 ml of isopropyl acetate and dried at 60°C/10mbar for 2 h to yield 1.40 g (70%) of pure (S)-2-methoxy-3-{4-[2-(5-methyl-2-phenyl-oxazol-4-yl)-ethoxy]-benzo[b]thiophen-7-yl}-propionic acid (Acid I) as a white crystals with a chemical purity of 99.8 % and an enantiomeric purity of >99.9%ee.

### Example 8.1

In a glove box (O₂ content ≤ 2 ppm), a 185- ml stainless steel autoclave was charged with 2.00 g of (Z)-2-methoxy-3-{4-[2-(5-methyl-2-phenyl-oxazol-4-yl)-ethoxy]-benzo[b]thiophen-7-yl}-acrylic acid (4.59 mmol), 4.48 mg of [Ir((S,S)-DBT-Bn-SIPHOX)(COD)]BARF (0.0023 mmol, S/C 2'000), 24 ml of methanol, 16 ml of tetrahydrofuran and 0.12 ml of (S)-1-phenylethylamine (0.93 mmol). The autoclave was sealed and the hydrogenation was run at 60°C for 20 h and subsequently 80°C for 2 h under 10 bar of hydrogen. After the autoclave was opened and the yellowish solution was rotary evaporated to dryness (50°C/5 mbar) to afford crude (S)-2-methoxy-3-{4-[2-(5-methyl-2-phenyl-oxazol-4-yl)-ethoxy]-benzo[b]thiophen-7-yl}-propionic acid (Acid I) as a white solid with a chemical purity of 98.9% (>99.9% conversion) and an enantiomeric purity of 99.6%.

### Example 8.2

In a glove box (O₂ content ≤ 2 ppm), a 185-ml stainless steel autoclave was charged with 2.00 g of (Z)-2-methoxy-3-{4-[2-(5-methyl-2-phenyl-oxazol-4-yl)-ethoxy]-benzo[b]thiophen-7-yl}-acrylic acid (4.59 mmol), 5.40 mg of [Ir((Sₐ,R)-DBT-Bn-SIPHOX)(COD)]BF₄ (0.0046 mmol, S/C 1'000), 24 ml of methanol, 16 ml of tetrahydrofuran and 0.12 ml of (S)-1-phenylethylamine (0.92 mmol). The autoclave was sealed and the hydrogenation was run at 60°C for 20 h and subsequently 80°C for 2 h under 10 bar of hydrogen. After the autoclave was opened and the yellowish solution was rotary evaporated to dryness (50°C/5 mbar) to afford crude (S)-2-methoxy-3-{4-[2-(5-methyl-2-phenyl-oxazol-4-yl)-ethoxy]-benzo[b]thiophen-7-yl}-propionic acid (Acid I) as a white solid with a chemical purity of 89.7% (91.1% conversion) and an enantiomeric purity of 99.8%.

### Example 8.3

In a glove box (O₂ content ≤ 2 ppm), a 185-ml stainless steel autoclave was charged with 2.00 g of (Z)-2-methoxy-3-{4-[2-(5-methyl-2-phenyl-oxazol-4-yl)-ethoxy]-benzo[b]thiophen-7-yl}-acrylic acid (4.59 mmol), 2.84 mg of [Ir((Rₐ,S)-DBT-Bn-SIPHOX)(COD)]OTf (0.0023 mmol, S/C 2'000), 24 ml of methanol, 16 ml of tetrahydrofuran and 0.12 ml of (S)-1-phenylethylamine (0.92 mmol). The autoclave was sealed and the hydrogenation was run at 60°C for 20 h and subsequently 80°C for 2 h under 30 bar of hydrogen. After the autoclave was opened and the yellowish solution was rotary evaporated to dryness (50°C/5 mbar) to afford crude (R)-2-methoxy-3-{4-[2-(5-methyl-2-phenyl-oxazol-4-yl)-ethoxy]-benzo[b]thiophen-7-yl}-propionic acid (Acid I) as a white solid with a chemical purity of 98.7% (99.7% conversion) and an enantiomeric purity of 99.8%.

### Example 8.4

In a glove box (O₂ content ≤ 2 ppm), a 185-ml stainless steel autoclave was charged with 2.00 g of (Z)-2-methoxy-3-{4-[2-(5-methyl-2-phenyl-oxazol-4-yl)-ethoxy]-benzo[b]thiophen-7-yl}-acrylic acid (4.59 mmol), 1.80 mg of [Ir((Sₐ,R)-DBT-Bn-SIPHOX)(COD)]BF₄ (0.0015 mmol, S/C 3'000), 24 ml of methanol, 16 ml of tetrahydrofuran and 0.12 ml of (S)-1-phenylethylamine (0.92 mmol). The autoclave was sealed and the hydrogenation was run at 60°C for 20 h and subsequently 80°C for 2 h under 60 bar of hydrogen. After the autoclave was opened and the yellowish solution was rotary evaporated to dryness (50°C/5 mbar) to afford crude (S)-2-methoxy-3-{4-[2-(5-methyl-2-phenyl-oxazol-4-yl)-ethoxy]-benzo[b]thiophen-7-yl}-propionic acid (Acid I) as a white solid with a chemical purity of 98.3% (99.4% conversion) and an enantiomeric purity of 99.7%.

### Example 8.5

In a glove box (O₂ content ≤ 2 ppm), a 185-ml stainless steel autoclave was charged with 0.77 mg of [Ir(COD)Cl]₂ (0.0012 mmol, S/C 2'000), 1.81 mg of (Rₐ,S)-DBT-Bn-SIPHOX (0.0023 mmol) and 10 ml of tetrahydrofuran. The formed yellow solution was stirred for 30 min at ambient temperature. Then, 2.00 g of (Z)-2-methoxy-3-{4-[2-(5-methyl-2-phenyl-oxazol-4-yl)-ethoxy]-benzo[b]thiophen-7-yl}-acrylic acid (4.59 mmol), 24 ml of methanol, 6 ml of tetrahydrofuran and 0.12 ml of (S)-1-phenylethylamine (0.92 mmol) were added. The autoclave was sealed and the hydrogenation was run at 60°C for 20 h and subsequently 80°C for 2 h under 30 bar of hydrogen. After the autoclave was opened and the yellowish solution was rotary evaporated to dryness (50°C/5 mbar) to afford crude (R)-2-methoxy-3-{4-[2-(5-methyl-2-phenyl-oxazol-4-yl)-ethoxy]-benzo[b]thiophen-7-yl}-propionic acid (Acid I) as a white solid with a chemical purity of 98.5% (>99.9% conversion) and an enantiomeric purity of 99.5%.

### Example 8.6

In a glove box (O₂ content ≤ 2 ppm), a 185-ml stainless steel autoclave was charged with 1.14 mg of [Ir(COD)₂]BF₄ (0.0023 mmol, S/C 2'000), 1.99 mg of (Rₐ,S)-DBT-Bn-SIPHOX (0.0025 mmol) and 10 ml of tetrahydrofuran. The formed yellow solution was stirred for 30 min at ambient temperature. Then, 2.00 g of (Z)-2-methoxy-3-{4-[2-(5-methyl-2-phenyl-oxazol-4-yl)-ethoxy]-benzo[b]thiophen-7-yl}-acrylic acid (4.59 mmol), 24 ml of methanol, 6 ml of tetrahydrofuran and 0.12 ml of (S)-1-phenylethylamine (0.92 mmol) were added. The autoclave was sealed and the hydrogenation was run at 60°C for 20 h and subsequently 80°C for 2 h under 30 bar of hydrogen. After the autoclave was opened and the yellowish solution was rotary evaporated to dryness (50°C/5 mbar) to afford crude (R)-2-methoxy-3-{4-[2-(5-methyl-2-phenyl-oxazol-4-yl)-ethoxy]-benzo[b]thiophen-7-yl}-propionic acid (Acid I) as a white solid with a chemical purity of 98.7% (>99.9% conversion) and an enantiomeric purity of 99.6%.

### Example 8.7

In a glove box (O₂ content ≤ 2 ppm), a 185-ml stainless steel autoclave was charged with 2.00 g of (Z)-2-methoxy-3-{4-[2-(5-methyl-2-phenyl-oxazol-4-yl)-ethoxy]-benzo[b]thiophen-7-yl}-acrylic acid (4.59 mmol), 1.80 mg of [Ir((Sₐ,R)-DBT-Bn-SIPHOX)(COD)]BF₄ (0.0015 mmol, S/C 3'000), 24 ml of methanol, 16 ml of tetrahydrofuran and 0.12 ml of (S)-1-phenylethylamine (0.92 mmol). The autoclave was sealed and the hydrogenation was run at 80°C for 22 h under 30 bar of hydrogen. After the autoclave was opened and the yellowish solution was rotary evaporated to dryness (50°C/5 mbar) to afford crude (S)-2-methoxy-3-{4-[2-(5-methyl-2-phenyl-oxazol-4-yl)-ethoxy]-benzo[b]thiophen-7-yl}-propionic acid (Acid I) as a white solid with a chemical purity of 96.7% (99.9% conversion) and an enantiomeric purity of 99.5%.

### Example 9

In an analogous manner to Example 4 the following hydrogenation was performed at 40°C under 30 bar of hydrogen (reaction time: 16 h) using [Ir((S,S)-Xyl-Skewphos)(COD)]BF₄ (S/C 1'000) as catalysts to afford crude (S)-2-methoxy-3-{4-[2-(5-methyl-2-phenyl-oxazol-4-yl)-ethoxy]-benzo[b]thiophen-7-yl}-propionic acid (Acid I) as a white solid with a chemical purity of 98.8 % (99.4% conversion) and an enantiomeric purity of 85%.

### Example 10

In an analogous manner to Example 2 the following hydrogenation was performed at 60°C under 30 bar of hydrogen (reaction time: 16 h) using [Ir((S,R,R)-Trifer) (COD)]BARF (S/C 250) as catalysts to afford crude (R)-2-methoxy-3-{4-[2-(5-methyl-2-phenyl-oxazol-4-yl)-ethoxy]-benzo[b]thiophen-7-yl}-propionic acid (Acid I) as a white solid with a chemical purity of 98.0 % (>99.9% conversion) and an enantiomeric purity of 86%.

## Claims

1. A process for the preparation of a compound of formula (I) 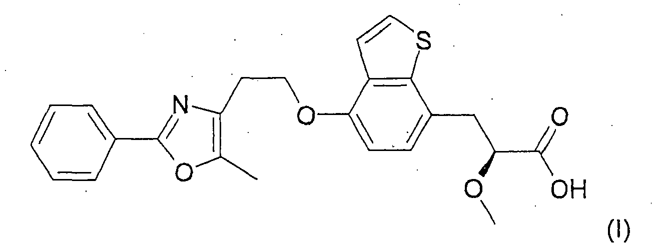 or a salt thereof, wherein a compound of formula (II) 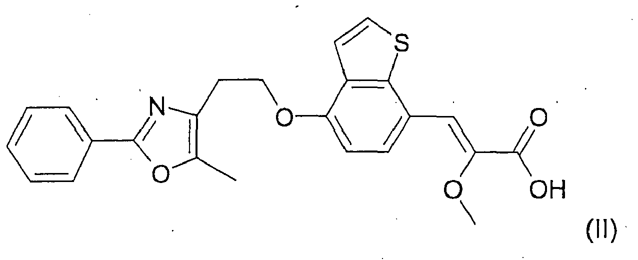 or a salt thereof is hydrogenated in the presence of a catalyst comprising iridium and a compound of formula (X) 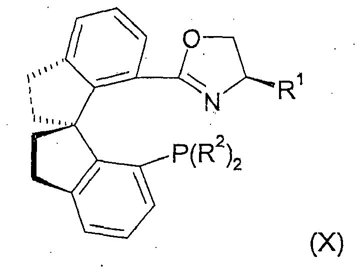 wherein
R¹ is alkyl, aryl or arylalkyl; and
R² is aryl.

2. A process according to claim 1, wherein R¹ is *iso*-propyl, phenyl or benzyl.

3. A process according to claim 1 or 2, wherein R² is phenyl, 3,5-di-methylphenyl or 3,5-di-*tert*-butyl-phenyl.

4. A process according to any one of claims 1 to 3, wherein the compound of formula (X) is
(Sₐ,R)-7-[4,5-Dihydro-4-benzyloxazol-2-yl]-7'-diphenylphosphino-1,1' spirobiindane;
(Sₐ,R)-7-[4,5-Dihydro-4-benzyloxazol-2-yl]-7'-di(3,5-dimethylphenyl)phosphino-1,1'-spirobiindane;
(Sₐ,R)-7-[4,5-Dihydro-4-benzyloxazol-2-yl]-7'-di(3,5-di-tert-butylphenyl) phosphino-1,1'-spirobiindane;
(Sₐ,R)-7-[4,5-Dihydro-4-phenyloxazol-2-yl]-7'-di(3,5-di-tert-butylphenyl) phosphino-1,1'-spirobiindane; or
(Sₐ,R)-7-[4,5-Dihydro-4-isopropyloxazol-2-yl]-7'-di(3,5-di-tert-butylphenyl) phosphino-1,1'-spirobiindane.

5. A process according to any one of claims 1 to 4, wherein the catalyst is Ir(L¹) (L²)ₙY
wherein
L¹ is a compound of formula (X) as defined in any one of claims 1 to 4;
L² is cyclooctene, 1,5-cyclooctadiene, ethylene, 1,5-hexadiene or norbornadiene;
Y is chloride, iodide, bromide, fluoride, trifluoroacetate, tetrafluoroborate, tetrakis[3,5-bis(trifluoromethyl)phenyl]borate, tetraphenylborate, hexafluoroantimonate, hexafluorophosphate, triflate, mesylate, perchlorate, perbromate, periodate, nitrate, hydrogene sulfate or acetylacetonate; and
n is 1 or 2.

6. A process according to any one of claims 1 to 5, wherein the catalyst is
[Ir((Sₐ,R)-7-[4,5-dihydro-4-benzyloxazol-2-yl]-7'-di(3,5-di-tert-butylphenyl) phosphino-1,1'-spirobiindane)(1,5-cyclooctadiene)][tetrakis[3,5-bis(trifluoromethyl)phenyl]borate];
[Ir((Sₐ,R)-7-[4,5-dihydro-4-benzyloxazol-2-yl]-7'-di(3,5-di-tert-butylphenyl) phosphino-1,1'-spirobiindane)(1,5-cyclooctadiene)][tetrafluoroborate];
[Ir((Sₐ,R)-7-[4,5-dihydro-4-benzyloxazol-2-yl]-7'-di(3,5-di-tert-butylphenyl) phosphino-1,1'-spirobiindane)(1,5-cyclooctadiene)] [trifluoromethanesulfonate]; or
[Ir((Sₐ,R)-7-[4,5-dihydro-4-benzyloxazol-2-yl]-7'-di(3,5-di-tert-butylphenyl) phosphino-1,1'-spirobiindane)(1,5-cyclooctadiene)][chloride].

7. The use of a catalyst as defined in any one of claims 1 to 6 for the preparation of a compound of formula (I) as defined in claims 1.

## Patentansprüche

1. Verfahren zur Herstellung einer Verbindung der Formel (I) 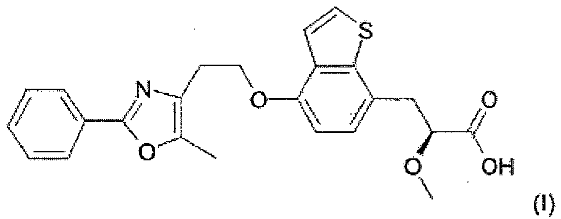 oder eines Salzes davon, wobei eine Verbindung der Formel (II) 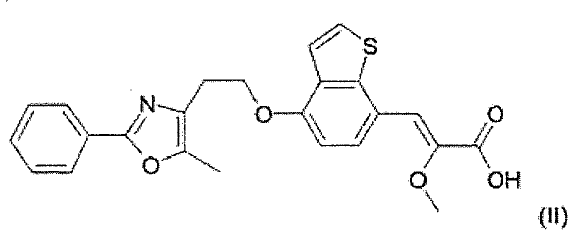 oder ein Salz davon hydriert wird in der Anwesenheit eines Katalysators, welcher Iridium und eine Verbindung der Formel (X) umfasst 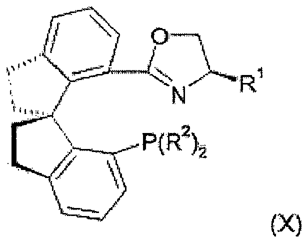 wobei
R¹ alkyl, aryl oder arylalkyl ist; und
R² aryl ist.

2. Verfahren nach Anspruch 1, wobei R¹ *iso*-propyl, phenyl oder benzyl ist.

3. Verfahren nach Anspruch 1 oder 2, wobei R² phenyl, 3,5-dimethylphenyl oder 3,5-di-*tert*-butyl-phenyl ist.

4. Verfahren nach einem der Ansprüche 1 bis 3, wobei die Verbindung der Formel (X)
(Sₐ,R)-7-[4,5-Dihydro-4-benzyloxazol-2-yl]-7'-diphenyl)phosphino-1,1'-spirobiindan;
(Sₐ,R)-7-[4,5-Dihydro-4-benzyloxazol-2-yl]-7'-di(3,5-di-methylphenyl)phosphino-1,1'-spirobiindan;
(Sₐ,R)-7-[4,5-Dihydro-4-benzyloxazol-2-yl]-7'-di(3,5-di-tert-butylphenyl)phosphino-1,1'-spirobiindan;
(Sₐ,R)-7-[4,5-Dihydro-4-phenyloxazol-2-yl]-7'-di(3,5-di-tert-butylphenyl) phosphino-1,1'-spirobiindan oder
(Sₐ,R)-7-[4,5-Dihydro-4-isopropyloxazol-2-yl]-7'-di(3,5-di-tert-butylphenyl) phosphino-1,1'-spirobiindan
ist.

5. Verfahren nach einem der Ansprüche 1 bis 4, wobei der Katalysator Ir(L¹)(L²)ₙY ist, wobei
L¹ eine Verbindung der Formel (X) ist wie in einem der Ansprüche 1 bis 4 definiert;
L² Cycloocten, 1,5-Cyclooctadien, Ethylen, 1,5-Hexadien oder Norbornadien ist;
Y Chlorid, Iodid, Bromid, Fluorid, Trifluoracetat, Tetrafluorborat, Tetrakis [3,5-bis(trifluormethyl)phenyl]borat, Tetraphenylborat, Hexafluorantimonat, Hexafluorphosphat, Triflat, Mesylat, Perchlorat, Perbromat, Periodat, Nitrat, Hydrogensulfat oder Acetylacetonat ist;
und n 1 oder 2 ist.

6. Verfahren nach einem der Ansprüche 1 bis 5, wobei der Katalysator
[Ir((Sₐ,R)-7-[4,5-Dihydro-4-benzyloxazol-2-yl]-7'-di(3,5-di-tert-butylphenyl) phosphin-1,1'-spirobiindan)(1,5-cyclooctadien)][tetrakis[3,5-bis (trifluormethyl)phenyl]Borat];
[Ir((Sₐ,R)-7-[4,5-Dihydro-4-benzyloxazol-2-yl]-7'-di(3,5-di-tert-butylphenyl) phosphino-1,1'-spirobiindan)(1,5-cyclooctadien)][Tetrafluorborat];
[Ir((Sₐ,R)-7-[4,5-Dihydro-4-benzyloxazol-2-yl]-7'-di(3,5-di-tert-butylphenyl) phosphino-1,1'-spirobiindan)(1,5-cyclooctadien)][Trifluormethansulfonat]; oder
[Ir((Sₐ,R)-7-[4,5-Dihydro-4-benzyloxazol-2-yl]-7'-di(3,5-di-tert-butylphenyl) phosphino-1,1'-spirobiindan)(1,5-cyclooctadien)][Chlorid]
ist.

7. Verwendung eines Katalysators wie in einem der Ansprüche 1 bis 6 definiert zur Herstellung einer Verbindung der Formel (I) wie in Anspruch 1 definiert.

## Revendications

1. Procédé pour la préparation d'un composé de formule (I) 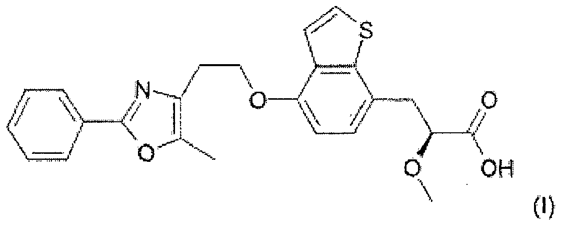 ou un sel de celui-ci, dans lequel un composé de formule (II) 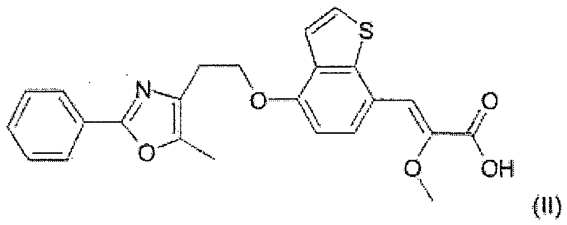 ou un sel de celui-ci est hydrogéné en présence d'un catalyseur comprenant de l'iridium et un composé de formule (X) 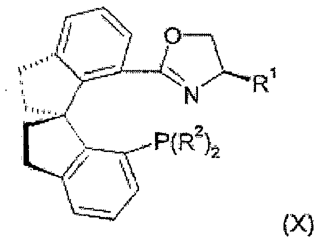 dans lequel
R¹ est alkyl, aryl ou arylalkyl ; et
R² est aryl.

2. Procédé selon la revendication 1, dans lequel R¹ est *iso*-propyl, phényl ou benzyl.

3. Procédé selon la revendication 1 ou 2, dans lequel R² est phényl, 3,5-diméthylphényl ou 3,5-di-*tert*-butyl-phényl.

4. Procédé selon l'une quelconque des revendications 1 à 3, dans lequel le composé de formule (X) est
(Sₐ,R)-7-[4,5-dihydro-4-benzyloxazol-2-yl]-7'-diphényl)phosphino-1,1'-spirobiindane;
(Sₐ,R)-7-[4,5-dihydro-4-benzyloxazol-2-yl]-7'-di(3,5-di-méthylphényl)phosphino-1,1'-spirobiindane;
(Sₐ,R)-7-[4,5-dihydro-4-benzyloxazol-2-yl]-7'-di(3,5-di-tert-butylphényl)phosphino-1,1'-spirobiindane;
(Sₐ,R)-7-[4,5-dihydro-4-phényloxazol-2-yl]-7'-di(3,5-di-tert-butylphényl) phosphino-1,1'-spirobiindane; ou
(Sₐ,R)-7-[4,5-dihydro-4-isopropyloxazol-2-yl]-7'-di(3,5-di-tert-butylphényl) phosphino-1,1'-spirobiindane.

5. Procédé selon l'une quelconque des revendications 1 à 4, dans lequel le catalyseur est Ir(L¹)(L²)ₙY
dans lequel
L¹ est un composé de formule (X) comme défini dans l'une quelconque des revendications 1 à 4 ;
L² est cyclooctène, 1,5-cyclooctadiène, éthylène, 1,5-hexadiène ou norbornadiène ;
Y est chlorure, iodure, bromure, fluorure, trifluoroacétate, tétrafluoroborate, tétrakis[3,5-bis(trifluorométhyl)phényl]borate, tétraphénylborate, hexafluoroantimoniate, hexafluorophosphate, triflate, mésylate, perchlorate, perbromate, périodate, nitrate, sulfate d'hydrogène or acétylacétonate;
et n est 1 ou 2.

6. Procédé selon l'une quelconque des revendications 1 à 5, dans lequel le catalyseur est
[Ir((Sₐ,R)-7-[4,5-dihydro-4-benzyloxazol-2-yl]-7'-di(3,5-di-tert-butylphényl) phosphino-1,1'-spirobiindane)(1,5-cyclooctadiène)][tétrakis[3,5-bis (trifluorométhyl)phényl] borate];
[Ir((Sₐ,R)-7-[4,5-dihydro-4-benzyloxazol-2-yl]-7'-di(3,5-di-tert-butylphényl) phosphino-1, 1'-spirobiindane)(1, 5-cyclooctadiène)][tétrafluoroborate];
[Ir((Sₐ,R)-7-[4,5-dihydro-4-benzyloxazol-2-yl]-7'-di(3,5-di-tert-butylphényl) phosphino-1,1'-spirobiindane)(1, 5-cyclooctadiène)][trifluorométhanesulfonate]; ou
[Ir((Sₐ,R)-7-[4,5-dihydro-4-benzyloxazol-2-yl]-7'-di(3,5-di-tert-butylphényl) phosphino-1,1'-spirobiindane)(1,5-cyclooctadiène)][chlorure].

7. Utilisation d'un catalyseur comme défini dans l'une quelconque des revendications 1 à 6 pour la préparation d'un composé de formule (I) comme défini dans la revendication 1.
